# EUROPEAN PATENT SPECIFICATION

(11) **EP 4 056 121 B1**
(45) Date of publication and mention of the grant of the patent: **15.05.2024**
(21) Application number: 21179114.0
(22) Date of filing: 11.06.2021
(51) Int. Cl.: A61B 6/03, A61N 5/10, A61B 6/00

(54) **ADAPTION OF A MEDICAL IMAGING PROCESS TO A SWALLOWING MOVEMENT OF A PATIENT**
ANPASSUNG EINES MEDIZINISCHEN BILDGEBUNGSVERFAHRENS AN EINE SCHLUCKBEWEGUNG EINES PATIENTEN
ADAPTATION D'UN PROCESSUS D'IMAGERIE MÉDICALE À UN MOUVEMENT DE DÉGLUTITION D'UN PATIENT

(43) Date of publication of application: 14.09.2022
(73) Proprietor: Siemens Healthineers AG, 91301 Forchheim (DE)
(72) Inventor: Baer-Beck, Matthias, 91080 Spardorf (DE); Hofmann, Christian, 91058 Erlangen (DE)
(74) Representative: Siemens Healthineers Patent Attorneys

(56) References cited:
- FR-A1- 2 839 894
- US-A1- 2012 043 475
- YOKO INAMOTO ET AL: "Evaluation of Swallowing Using 320-detector-row Multislice CT. Part II: Kinematic Analysis of Laryngeal Closure during Normal Swallowing", DYSPHAGIA, SPRINGER-VERLAG, NE, vol. 26, no. 3, 5 March 2010 (2010-03-05), pages 209-217, XP019949915, ISSN: 1432-0460, DOI: 10.1007/S00455-010-9276-2

## Description

The invention relates to a method for CT imaging of an examination area influenced by a swallowing movement of a patient. The invention also relates to a method for radiation treatment planning. Further, the invention relates to a CT image reconstruction device. Additionally, the invention relates to a medical imaging system. Furthermore, the invention relates to a medical radiation treatment planning device. Beyond that, the invention relates to a medical radiation treatment system.

In radiation therapy it is crucial that a motion of a patient as well as organ motion is considered during treatment planning. The motion of a patient can be conscious, for example breathing or swallowing, or unconscious, for example organ motion.

Reference is made to US 2012/043475 A1, FR 2 839 894 A1 and YOKO INAMOTO ET AL: "Evaluation of Swallowing Using 320-detector-row Multislice CT. Part II: Kinematic Analysis of Laryngeal Closure during Normal Swallowing", DYSPHAGIA, SPRINGER-VERLAG, NE, vol. 26, no. 3, 5 March 2010 (2010-03-05), pages 209-217.

Current respiratory motion is for example considered during treatment planning. This leads to higher accuracy and reduced dose to organs at risk and healthy tissue.

For tumors in the head and neck region motion originating from swallowing is currently not compensated for as it is done for respiratory motion. There are no scan modes or reconstruction algorithms available that can account or correct for swallowing motion.

The motion due to swallowing is non-negligible and currently causes significant errors in radiation plans, respectively causes significant dose to healthy tissue or organs at risk. The patient will swallow during the treatment and there is currently no way to account for that.

In today's radiation therapy planning, swallowing is not considered directly, but typically the margins around the tumor are simply enlarged to consider the possible movement of the tumor due to swallowing. The drawback of this workaround is the potential higher dose to healthy tissue or organs at risk, for example brain, salivary glands, or spinal cord in the case of head and neck. A further drawback of the enlarge-margins approach is that it is not adaptive: If the swallowing motion is - for whatever reason - different during planning and treatment, the margins cannot be adapted and either even more healthy tissue is radiated or at least a part of the tumor may move out of the treatment beam.

Hence, a problem underlying to the invention is to improve the planning of a radiation process such that the safety of the patient and the reliability of treatment is improved.

That problem is solved by a method for CT imaging of an examination area influenced by a swallowing movement of a patient according to claim 1, by a method for radiation treatment planning according to claim 6, by a CT image reconstruction device according to claim 7, by a medical imaging system according to claim 8, by a medical radiation treatment planning device according to claim 9 and by a medical radiation treatment system according to claim 10.

According to the method for CT imaging of an examination area influenced by a swallowing movement of a patient, CT raw data are recorded from an examination area of the patient. Further, different movement phases of the swallowing movement of the patient are detected. The detection of the swallowing movement and in particular of the different movement phases can be realized based on the acquired CT raw data, it can also be attained by using additional sensor data, preferably image data of the patient. Based on the detected movement phases, different raw data sets of the recorded CT raw data assigned to different detected movement phases are detected. For each of the determined raw data sets an individual CT image assigned to an individual movement phase is reconstructed.

Advantageously, the quality of medical images can be improved and the operating expense and time for wasted scan processes can be reduced. Hence, the medical imaging resources can be used more effectively. Further, the comfort for the patient is increased, since the number of additional scan attempts and the time for these attempts can be reduced. Furthermore, the radiation exposure due to additional scan attempts can also be reduced. Furthermore, the images of different swallowing phases can be used for defining a time dependent treatment area for radiation treatment planning. Since the treatment area is determined more accurately, the radiation dose can be reduced and healthy tissue and organs at risk are better protected during treatment.

In the method for radiation treatment planning according to the invention, image data are recorded using the method for CT imaging of an examination area influenced by a swallowing movement of a patient according to the invention. Then, the recorded image data are evaluated for determining an expectable dynamic position of a treatment area of a patient, which is part of the examination area. Advantageously, the dynamic position of the treatment area can be calculated in advance and the radiation can be restricted to an area at the dynamic position. If the method is used during a treatment, i.e. for controlling the radiation of a treatment area, a radiation can also be stopped, when a swallowing movement is detected. Hence, a gated treatment is realized for radiation, which is prone to swallowing motion.

The CT image reconstruction device according to the invention comprises a raw data interface for recording CT raw data of an examination area of a patient. Further, the CT image reconstruction device includes a swallowing movement detection unit for detecting different movement phases of a swallowing movement of the patient. Part of the CT image reconstruction device is also an assigning unit for determining different raw data sets of the recorded CT raw data assigned to different detected movement phases, based on the detected movement phases and a reconstruction unit for reconstructing for each of the determined raw data sets an individual CT image assigned to an individual movement phase. The CT image reconstruction device shares the advantages of the method for CT imaging of an examination area influenced by a swallowing movement of a patient according to the invention.

The medical imaging system comprises a CT scan unit for carrying out a medical imaging from a patient and the CT image reconstruction device according to the invention. The medical imaging system shares the advantages of the CT image reconstruction device according to the invention.

The medical radiation treatment planning device comprises a medical imaging system for recording image data using the method for CT imaging of an examination area influenced by a swallowing movement of a patient and a planning unit for evaluating the recorded image data for determining an expectable dynamic position of radiation area of a patient. The medical radiation treatment planning device shares the advantage of the CT image reconstruction device.

The medical radiation treatment system comprises a medical radiation treatment planning device according to the invention. Further, the medical radiation treatment system includes a radiation treatment unit for radiating a radiation area of a patient based on the determined expectable dynamic position. As above-mentioned, the medical radiation treatment system advantageously takes into account the swallowing motion of a patient during the treatment such that healthy tissue and organs at risk are protected during treatment. The protection can be achieved by adapting the position of the treatment area to the swallowing movement or by restriction of the treatment time intervals to time intervals between the swallowing movement.

The essential components of the CT image reconstruction device according to the invention and the medical radiation treatment planning device according to the invention, can for the most part be designed in the form of software components and if required, by further adding some hardware as for example a surrogate monitoring unit like a camera. This applies in particular to the swallowing movement detection unit, the assigning unit, the reconstruction unit and the planning unit, but also parts of the input interfaces. In principle, however, some of these components can also be implemented in the form of software-supported hardware, for example FPGAs or the like, especially when it comes to particularly fast calculations. Likewise, the required interfaces, for example if it is only a matter of transferring data from other software components, can be designed as software interfaces. However, they can also be designed as hardware-based interfaces that are controlled by suitable software. Furthermore, some parts of the above-mentioned components may be distributed and stored in a local or regional or global network or a combination of a network and software, in particular a cloud system.

A largely software-based implementation has the advantage that medical imaging systems or treatment systems that have already been used, can easily be retrofitted by a software update in order to work in the manner according to the invention. In this respect, the object is also achieved by a corresponding computer program product with a computer program that can be loaded directly into a memory device of for example a control device of a medical imaging system or treatment system, with program sections, in order to carry out all steps of the methods according to the invention, if the program is executed in the medical imaging system or treatment system, in particular the control device. In addition to the computer program, such a computer program product may contain additional components such as a documentation and/ or additional components, including hardware components such as Hardware keys (dongles etc.) for using the software.

For transport to the CT image reconstruction device and/or the medical radiation treatment planning system and/ or for storage on or in the CT image reconstruction device and/or the medical radiation treatment planning system, a computer-readable medium, for example a memory stick, a hard disk or some other transportable or permanently installed data carrier is used on which the program sections of the computer program that can be read in and executed by a computer unit of the CT image reconstruction device and/or the medical radiation treatment planning system are stored. The computer unit can comprise for example, one or more cooperating microprocessors or the like used for this purpose.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the method according to the invention the swallowing movement is detected based on a swallowing surrogate. A surrogate has to be understood as a physiological process, which can be detected using sensor technology and which is correlated to the actual physiological process to be monitored. Hence, the actual interesting physiological process, in the present case the movement of an examination area or a treatment area due to the swallowing behaviour of a patient, is indirectly monitored.

The swallow surrogate can be either measured directly by an external device, for example a camera or a surface tracking device, or it can be determined indirectly by an image-based approach. In case of a direct measurement using an external device, the imaging of the swallowing motion can be done in analogy to the case of 4D phase correlated reconstruction for respiratory motion.

In a further variant of the method according to the invention, the swallowing surrogate is monitored during the acquisition of CT raw data. Preferred, the swallowing surrogate is monitored using an optical 3D-camera. In that variant, an additional monitoring unit is used additionally to the CT acquisition unit. Monitoring the swallowing movement can be used for determining the time intervals of swallowing movements and for assigning CT raw data to phases of movements and phases of rest of the patient.

In the method according to the invention, an image metric is defined, which is sensitive and specific to the swallowing movement, the different movement phases are assigned to different values of the image metric and the raw data sets are determined and assigned to different movement phases based on different values of the image metric. A typical metric for motion detection is for example the image entropy. Using the entropy a metric for motion is described in Doil Kim, Jiyoung Choi, Duhgoon Lee, Hyesun Kim, Jiyoung Jung, Minkook Cho & Kyoung-Yong Lee, "Motion correction for routine X-ray lung CT imaging", Nature Scientific Reports 11 (3695), 2021.

For recording the swallowing movement, a continuous data acquisition over several swallowing cycles is performed. In a first step, the motion metric can be used to detect the time frames where the swallowing occurs. For this purpose, images are reconstructed without making any assumptions on the swallowing motion. Hence, an image is reconstructed every 360° angle segment of the acquisition, wherein the acquisition is carried out continuously over several swallowing cycles.

Based on the acquired data, the motion metric is evaluated in reconstructed images to detect the images or time frames where the motion occurs. Depending on the scan parameters, it may be possible to distinguish between different motion phases. For example, a large value of the metric is assigned to a strong motion, a lower value of the metric is assigned to a weak motion and a minimum value is assigned to a no-motion phase. Once, one has determined the time frames for the different motion phases, one can start to optimize the data range that is used to reconstruct the individual images of the swallowing cycle assigned to different metrics and motion phases. The data for each phase are assembled from data ranges distributed over the whole acquisition, i.e. for several swallowing cycles, so that the motion metric of the final reconstructed phase image reaches a minimum.

To assure that for each z-position a complete "swallow" cycle is acquired, a dedicated scan mode can be defined. One can use "swallowing" commands in analogy to breathing commands to synchronize the acquisition with the swallowing commands and the swallowing movement. The swallow commands need to be performed several times depending on the collimation of the system and the z-range to be scanned, so that for z-position a complete swallowing cycle is acquired. The acquisition can be realized using low dose technology. Hence, the swallowing movement is divided into different swallowing phases and for each z-position enough CT raw data are acquired to be able to reconstruct an image for each swallowing phase at each z-position.

The invention is explained below with reference to the figures enclosed once again. The same components are provided with identical reference numbers in the various figures.

The figures are usually not to scale.
FIG 1 shows a CT-system according to an embodiment of the invention,
FIG 2 shows a flow chart diagram illustrating the method of CT imaging of an examination area influenced by a swallowing movement of a patient according to an embodiment of the invention,
FIG 3 shows a flow chart diagram illustrating the method for CT imaging of an examination area influenced by a swallowing movement of a patient according to an alternative embodiment of the invention,
FIG 4 shows a flow chart diagram illustrating a further alternative embodiment of the method for CT imaging of an examination area influenced by swallowing movements of a patient,
FIG 5 shows a flow chart diagram illustrating a method for radiation treatment planning according to an embodiment of the invention,
FIG 6 shows a schematic view on a CT image reconstruction device according to an embodiment of the invention,
FIG 7 shows a schematic view on a medical radiation treatment system according to an embodiment of the invention.

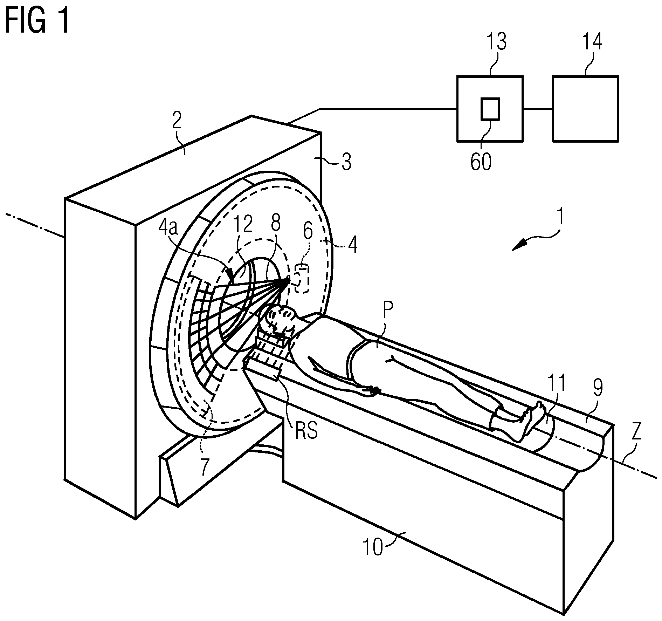

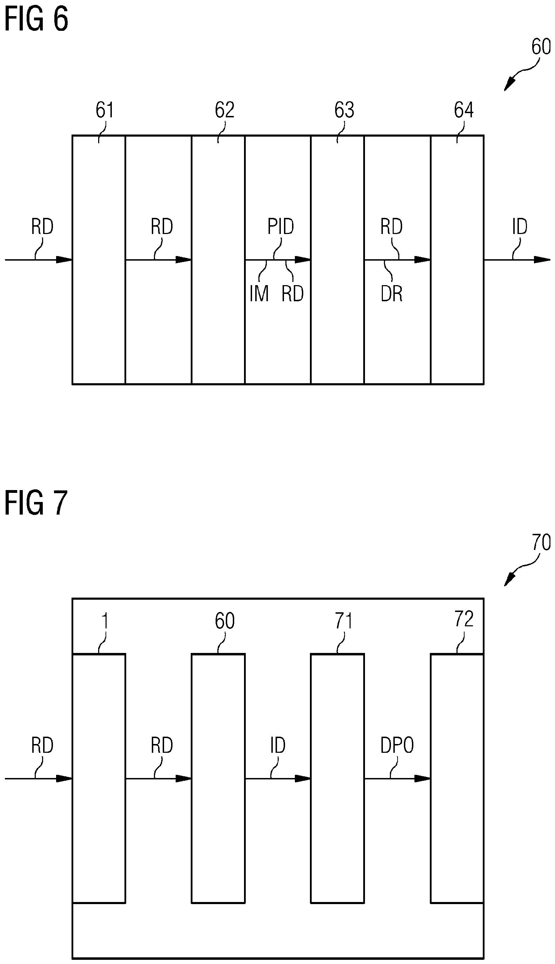

FIG 1 shows a schematic representation of a computer tomography system 1 comprising a CT image reconstruction device 60 according to an embodiment of the invention as later discussed in detail in context with FIG 6 and FIG 7. The arrangement comprises a gantry also called as scan unit 2 with a stationary part 3, also referred to as a gantry frame, and with a part 4, which can be rotated about a system axis, also referred to as a rotor or drum. The rotating part 4 has an imaging system (X-ray system) 4a, which comprises an X-ray source 6 and an X-ray detector 7, which are arranged on the rotating part 4 opposite one another. The X-ray source 6 and the X-ray detector 7 form the imaging system 4a. When the computer tomography system 1 is in operation, the X-ray source 6 emits X-rays 8 in the direction of the X-ray detector 7, penetrates a measurement object P, for example a patient P, and is transmitted by the X-ray detector 7 in the form of measurement data or measurement signals recorded.

In FIG 1, a patient table 9 for positioning the patient P can also be seen. The patient table 9 comprises a bed base 10, on which a patient support plate 11, which is provided for actually positioning the patient P, is arranged. The patient support plate 11 can be adjusted relative to the bed base 10 in the direction of the system axis z, i.e. in the z direction, so that it enters an opening 12 such that the patient P can be introduced into the opening 12 of the scan unit 2 for recording X-ray projections from the patient P. A computational processing of the X-ray projections recorded with the imaging system 4a or the reconstruction of sectional images, 3D images or a 3D data set based on the measurement data or measurement signals of the X-ray projections is carried out in an image computer 13 of the computer tomography system 1. The sectional images or 3D images can be displayed on a display device 14. The image computer 13 also comprises the CT image reconstruction device 60 according to an embodiment of the invention. The image computer 13 can also be designed as a control unit for controlling an imaging process for controlling the scan unit 2 and in particular the imaging system 4a of the scan unit 2. In FIG 1, also a sensor device, which works as a surrogate monitoring unit RS, is depicted. The surrogate monitoring unit RS emits radar waves (symbolised as dashed lines in FIG 1) to the area to be scanned, which is the neck of the patient P.

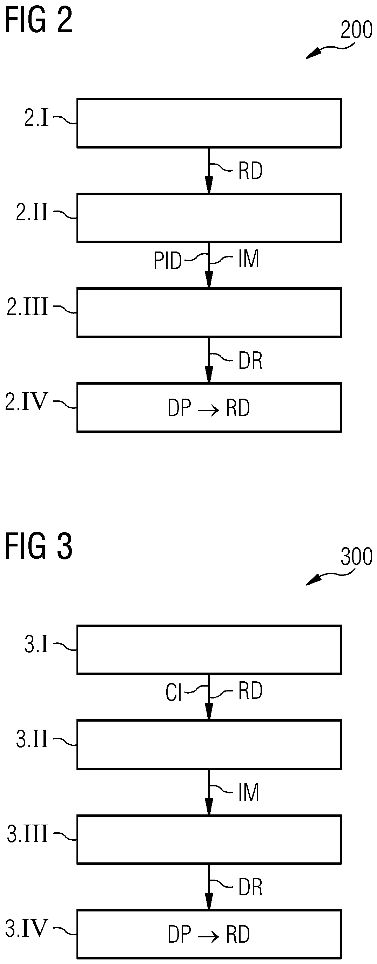

FIG 2 shows a flow chart diagram 200, which illustrates the method for CT imaging of an examination area influenced by swallowing movements of a patient.

In step 2.I, CT raw data RD of an examination area, i.e. the neck portion of a patient are acquired.

In step 2.II different movement phases of the swallowing movement are detected. For this purpose, a provisional reconstruction based on the acquired raw data RD is carried out. The provisional image data PID are reconstructed without making any assumptions on the swallowing motion. For example, one image is reconstructed every 360° angle segment of the raw data acquisition. Hence, the acquisition is continuously performed over several swallowing cycles.

Then, in the reconstructed provisional image data PID an image metric IM is detected, which is sensitive and specific to swallowing motion. A typical image metric for motion detection is for example the image entropy. The motion metric is then used to detect the images or time frames where the swallowing motion occurs.

Depending on the value of the scan parameters, it may be possible to distinguish between different motion phases, for example strong motion = large value of the metric, weak motion = lower value of the metric and no-motion = minimum value for the metric.

After having determined the time frames for the different motion phases, in step 2.III, the data range DR for individual images of different phases is determined. In other words, for each motion phase, raw data RD from several swallowing cycles are assigned to each other for reconstruction in step 2.IV so that the motion metric of the final reconstructed phase images reaches a minimum.

In step 2.IV, image data ID are reconstructed, wherein for each different motion phase, the data portions DP of raw data RD assigned to an individual motion phase are used for reconstruction.

In FIG 3 a flow chart diagram 300 is depicted, which illustrates the method for CT imaging of an examination area influenced by swallowing movements of a patient according to an alternative embodiment of the invention.

In step 3.I, CT raw data RD of an examination area, i.e. the neck portion of a patient are acquired. Simultaneously, a swallow surrogate is measured directly by a 3D camera.

In step 3.II, based on the camera images CI, different movement phases of the swallowing movement are detected. For that purpose, for each frame of raw data, an image metric IM is detected based on the camera images CI, which is sensitive and specific to swallowing motion. A typical image metric IM for motion detection is for example the image entropy. The motion metric is then used to detect the images or time frames where the swallowing motion occurs.

Thereby depending on the value of the scan parameters, it may be possible to distinguish between different motion phases, for example strong motion = large value of the metric, weak motion = lower value of the metric and no-motion = minimum value for the metric.

After having determined the time frames for the different motion phases, in step 3.III, the data ranges DR for individual images of different phases are determined. In other words, for each motion phase, raw data RD from several swallowing cycles are assigned to each other for reconstruction in step 3.IV so that the motion metric of the final reconstructed phase images reaches a minimum.

In step 3.IV, image data ID are reconstructed, wherein for each different motion phase, the data portions DP of raw data RD assigned to an individual motion phase are used for reconstruction.

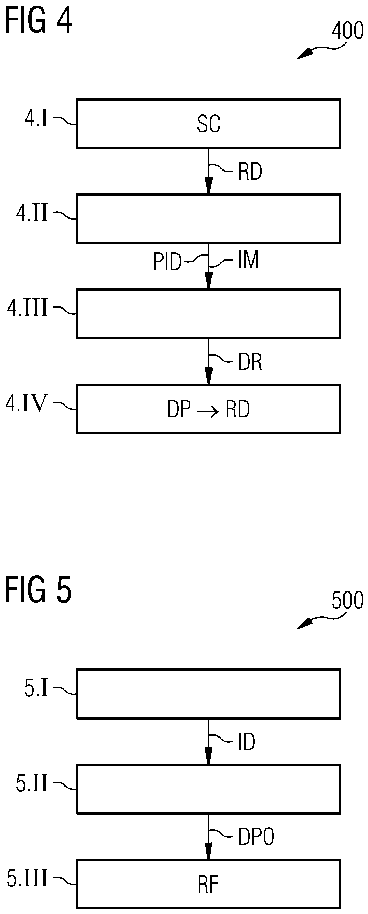

In FIG 4 a flow chart diagram 400 is illustrated for a detailed description of a further alternative embodiment of the method for CT imaging of an examination area influenced by swallowing movements of a patient.

In step 4.I CT raw data RD of an examination area, i.e. the neck portion of a patient are acquired. During the acquisition, for each z-position a swallowing command SC is given to synchronize the acquisition with the swallowing event of the patient, since it has to be assured that for each z-position, raw data, concerning a complete "swallow" cycle, are acquired. These swallow commands SC need to be performed several times depending on the collimation of the system and the z-range to be scanned so that for each z-position, raw data RD for a complete swallowing cycle with the above-mentioned movement phases are acquired.

Steps 4.II to 4.IV are then the same as in the first embodiment related to FIG 2.

In FIG 5, a flow chart 500 is depicted, which illustrates a method for radiation treatment planning according to an embodiment of the invention.

In step 5.I, image data ID are recorded using the method for CT imaging of an examination area influenced by swallowing movements of a patient according to one of the embodiments illustrated in FIG 2 to FIG 4.

Then, in step 5.II, based on the recorded image data ID the movement and dynamic position DPO of a target area, for example an organ in the neck, is determined.

Based on the knowledge acquired in step 5.II, the region RF to be radiated is determined based on the dynamic position DP of the target area in step 5.III.

In FIG 6, a CT image reconstruction device 60 according to an embodiment of the invention is schematically illustrated. The CT image reconstruction device 60 comprises a raw data interface 61 for recording CT raw data RD of an examination area of a patient. Further, the CT image reconstruction device 60 includes a swallowing movement detection unit 62 for detecting different movement phases of the swallowing movement of a patient. The swallowing movement detection unit 62 reconstructs provisional image data PID without making any assumptions on the swallowing motion. For example, one image is reconstructed every 360° angle segment of the raw data acquisition. Hence, the acquisition is continuously performed over several swallowing cycles. Then, in the reconstructed provisional image data PID an image metric IM is detected, which is sensitive and specific to swallowing motion. As mentioned above, a typical image metric for motion detection is for example the image entropy. The motion metric is then used to detect the images or time frames where the swallowing motion occurs.

Furthermore, the CT image reconstruction device 60 comprises an assigning unit 63 for determining different raw data sets of the recorded CT raw data assigned to different detected movement phases based on the detected movement phases. In the assigning unit 63, data ranges DR for individual images of different phases are determined. In other words, for each motion phase, raw data from several swallowing cycles are used for reconstruction so that the motion metric of the final reconstructed phase images reaches a minimum.

The CT image reconstruction device 60 also includes a reconstruction unit 64 for reconstructing for each of the determined raw data sets an individual CT image ID assigned to an individual movement phase.

In FIG 7, a medical radiation treatment system 70 is schematically illustrated. The medical radiation treatment system 70 comprises a CT system 1 for recording CT raw data RD, which are used as basis for a planning of a radiation treatment of a patient. Further, the medical radiation treatment system 70 comprises a CT image reconstruction device 60 as depicted in FIG 6, for reconstructing image data ID based on the raw data RD acquired by the CT system 1.

Furthermore, the medical radiation treatment system 70 comprises a medical radiation treatment planning unit 71 for evaluating the recorded image data ID for determining an expectable dynamic position DPO of radiation area of a patient. The medical radiation treatment system 70 also comprises a radiation treatment unit 72 for radiating a radiation area of a patient based on the determined expectable dynamic position.

The use of the undefined article "a" or "one" does not exclude that the referred features can also be present several times. Likewise, the term "unit" or "device" does not exclude that it consists of several components, which may also be spatially distributed.

## Claims

1. Method for CT imaging of an examination area influenced by a swallowing movement of a patient (P), comprising the steps of:
- recording CT raw data (RD) of the examination area,
- detecting different movement phases of the swallowing movement, wherein an image metric (IM) is defined, which is sensitive and specific to the swallowing movement, and the different movement phases are assigned to different values of the image metric (IM),
- determining different raw data sets of the recorded CT raw data (RD) assigned to different detected movement phases based on the detected movement phases, wherein the raw data sets (RD) are determined and assigned to different movement phases based on different values of the image metric (IM),
- reconstructing for each of the determined raw data sets an individual CT image (ID) assigned to an individual movement phase.

2. Method according to claim 1, wherein the swallowing movement is detected based on a swallowing surrogate.

3. Method according to claim 2, wherein the swallowing surrogate is monitored during the acquisition of CT raw data (RD).

4. Method according to claim 3, wherein the swallowing surrogate is monitored using an optical camera.

5. Method according to one of the preceding claims, wherein the swallowing movement is divided into different swallowing phases and for each z-position enough CT raw data (RD) are acquired to be able to reconstruct an image (ID) for each swallowing phase at each z-position.

6. Method for radiation treatment planning, comprising:
- recording image data (ID) using the method according to anyone of claims 1 to 5,
- evaluating the recorded image data (ID) for determining an expectable dynamic position (DP) of a radiation area of a patient (P).

7. CT image reconstruction device (60), comprising:
- a raw data interface (61) for recording CT raw data (RD) of an examination area of a patient (P),
- a swallowing movement detection unit (62) for detecting different movement phases of a swallowing movement of the patient (P), wherein an image metric (IM) is defined, which is sensitive and specific to the swallowing movement, and the different movement phases are assigned to different values of the image metric (IM),
- an assigning unit (63) for determining different raw data sets of the recorded CT raw data (RD) assigned to different detected movement phases, based on the detected movement phases, wherein the raw data sets (RD) are determined and assigned to different movement phases based on different values of the image metric (IM),
- a reconstruction unit (64) for reconstructing for each of the determined raw data sets an individual CT image (ID) assigned to an individual movement phase.

8. Medical imaging system (1), comprising:
- a CT scan unit (2) for carrying out a medical imaging from a patient (P),
- a CT image reconstruction device (60) according to claim 7.

9. Medical radiation treatment planning device (71), comprising:
- the medical imaging system (1) according to claim 8 for recording image data (ID) using the method according to anyone of claims 1 to 6,
- a planning unit (71) for evaluating the recorded image data (ID) for determining an expectable dynamic position (DPO) of a radiation area of a patient (P).

10. Medical radiation treatment system (70), comprising:
- a medical radiation treatment planning device (71) according to claim 9,
- a radiation treatment unit (72) for radiating a radiation area of a patient (P) based on the determined expectable dynamic position (DPO).

11. Computer program product with a computer program, which can be loaded directly into a memory device of a medical imaging system (1) or a medical radiation treatment planning device (71), with program sections to perform all the steps of anyone of the claims 1 to 6, when the computer program is carried out in the medical imaging system (1) or the medical radiation treatment planning device (71).

12. Computer readable medium, on which program sections that can be read in and executed by a computer unit are stored in order to carry out all steps of the methods according to anyone of the claims 1 to 6, when the program sections are executed by the computer unit.

## Patentansprüche

1. Verfahren zur CT-Bildgebung eines Untersuchungsbereichs, der durch eine Schluckbewegung eines Patienten (P) beeinflusst wird, umfassend die Schritte:
- Aufzeichnen von CT-Rohdaten (Raw Data, RD) des Untersuchungsbereichs,
- Detektieren unterschiedlicher Bewegungsphasen der Schluckbewegung, wobei eine Bildmetrik (Image Metric, IM) definiert ist, die empfindlich und spezifisch für eine Schluckbewegung ist, und die unterschiedlichen Bewegungsphasen unterschiedlichen Werten der Bildmetrik (IM) zugewiesen sind,
- Bestimmen unterschiedlicher Rohdatensätze der aufgezeichneten CT-Rohdaten (RD), die, basierend auf den detektierten Bewegungsphasen, unterschiedlichen detektierten Bewegungsphasen zugewiesen sind, wobei die Rohdatensätze (RD) basierend auf unterschiedlichen Werten der Bildmetrik (IM) bestimmt und unterschiedlichen Bewegungsphasen zugewiesen werden,
- Rekonstruieren, für jeden der bestimmten Rohdatensätze, eines einzelnen CT-Bildes (ID), das einer einzelnen Bewegungsphase zugewiesen ist.

2. Verfahren gemäß Anspruch 1, wobei die Schluckbewegung basierend auf einem Schlucksurrogat detektiert wird.

3. Verfahren gemäß Anspruch 2, wobei das Schlucksurrogat während der Erfassung von CT-Rohdaten (RD) überwacht wird.

4. Verfahren gemäß Anspruch 3, wobei das Schlucksurrogat unter Verwendung einer optischen Kamera überwacht wird.

5. Verfahren gemäß einem der vorstehenden Ansprüche, wobei die Schluckbewegung in unterschiedliche Schluckphasen unterteilt ist und für jede z-Position genügend CT-Rohdaten (RD) erfasst werden, um ein Bild (ID) für jede Schluckphase an jeder z-Position rekonstruieren zu können.

6. Verfahren zur Strahlungsbehandlungsplanung, umfassend:
- Aufzeichnen von Bilddaten (Image Data, ID) unter Verwendung des Verfahrens gemäß einem der Ansprüche 1 bis 5,
- Auswerten der aufgezeichneten Bilddaten (ID) zum Bestimmen einer zu erwartenden dynamischen Position (DP) eines Strahlungsbereichs eines Patienten (P).

7. CT-Bild-Rekonstruktionsvorrichtung (60), umfassend:
- eine Rohdatenschnittstelle (61) zum Aufzeichnen von CT-Rohdaten (RD) eines Untersuchungsbereichs eines Patienten (P),
- eine Schluckbewegungsdetektionseinheit (62) zum Detektieren unterschiedlicher Bewegungsphasen einer Schluckbewegung des Patienten (P), wobei eine Bildmetrik (IM) definiert ist, die empfindlich und spezifisch für die Schluckbewegung ist, und die unterschiedlichen Bewegungsphasen unterschiedlichen Werten der Bildmetrik (IM) zugewiesen sind,
- eine Zuweisungseinheit (63) zum Bestimmen unterschiedlicher Rohdatensätze der aufgezeichneten CT-Rohdaten (RD), die, basierend auf den detektierten Bewegungsphasen unterschiedlichen detektierten Bewegungsphasen zugewiesen sind, wobei die Rohdatensätze (RD) basierend auf unterschiedlichen Werten der Bildmetrik (IM) bestimmt und unterschiedlichen Bewegungsphasen zugewiesen werden,
- eine Rekonstruktionseinheit (64) zum Rekonstruieren, für jeden der bestimmten Rohdatensätze, eines einzelnen CT-Bildes (ID), das einer einzelnen Bewegungsphase zugewiesen ist.

8. Medizinisches Bildgebungssystem (1), umfassend:
- eine CT-Abtasteinheit (2) zum Ausführen einer medizinischen Bildgebung von einem Patienten (P),
- eine CT-Bild-Rekonstruktionsvorrichtung (60) gemäß Anspruch 7.

9. Medizinische Vorrichtung zur Strahlungsbehandlungsplanung (71), umfassend:
- das medizinische Bildgebungssystem (1) gemäß Anspruch 8 zum Aufzeichnen von Bilddaten (ID) unter Verwendung des Verfahrens gemäß einem der Ansprüche 1 bis 6,
- eine Planungseinheit (71) zum Auswerten der aufgezeichneten Bilddaten (ID) zum Bestimmen eines zu erwartenden dynamischen Position (DPO) eines Strahlungsbereichs eines Patienten (P).

10. Medizinisches Strahlungsbehandlungssystem (70), umfassend:
- eine Vorrichtung zur Strahlungsbehandlungsplanung (71) gemäß Anspruch 9,
- eine Strahlungsbehandlungseinheit (72) zum Bestrahlen eines Strahlungsbereichs eines Patienten (P) basierend auf der bestimmten zu erwartenden dynamischen Position (DPO).

11. Computerprogrammprodukt mit einem Computerprogramm, das direkt in eine Speichervorrichtung eines medizinischen Bildgebungssystems (1) oder einer medizinischen Vorrichtung zur Strahlungsbehandlungsplanung (71) geladen werden kann, mit Programmabschnitten zum Durchführen aller Schritte eines der Ansprüche 1 bis 6, wenn das Computerprogramm in dem medizinischen Bildgebungssystem (1) oder der medizinischen Vorrichtung zur Strahlungsbehandlungsplanung (71) ausgeführt wird.

12. Computerlesbares Medium, auf dem Programmabschnitte, die von einer Computereinheit eingelesen und ausgeführt werden können, gespeichert sind, um alle Schritte der Verfahren gemäß einem der Ansprüche 1 bis 6 auszuführen, wenn die Programmabschnitte von der Computereinheit ausgeführt werden.

## Revendications

1. Procédé d'imagerie CT d'une zone à examiner influencée par un mouvement de déglutition d'un patient (P), comprenant les stades de :
- enregistrement de données (RD) brutes CT de la zone à examiner,
- détection de phases de mouvement différentes du mouvement de déglutition, dans lequel on définit une métrique (IM) d'image, qui est sensible et spécifique au mouvement de déglutition et on affecte les différentes phases de mouvement à des valeurs différentes de la métrique (IM) d'image,
- détermination de différents ensembles de données brutes des données (RD) brutes CT enregistrées affectés à des phases de mouvement différentes détectées sur la base des phases de mouvement détectées, dans lequel on détermine les ensembles (RD) de données brutes et on les affecte à des phases de mouvement différentes sur la base de valeurs différentes de la métrique (IM) d'image,
- reconstruction pour chacun des ensembles de données brutes déterminés d'une image (ID) CT individuelle affectée à une phase de mouvement individuelle.

2. Procédé suivant la revendication 1, dans lequel on détecte le mouvement de déglutition sur la base d'un substitut de déglutition.

3. Procédé suivant la revendication 2, dans lequel on contrôle le substitut de déglutition pendant l'acquisition des données (RD) brutes CT.

4. Procédé suivant la revendication 3, dans lequel on contrôle le substitut de déglutition en utilisant un appareil photographique optique.

5. Procédé suivant l'une des revendications précédentes, dans lequel on subdivise le mouvement de déglutition en différentes phases de déglutition et on acquiert pour chaque position z suffisamment de données (RD) brutes CT, pour être à même de reconstruire une image (ID) pour chaque phase de déglutition à chaque position z.

6. Procédé de planification d'un traitement par rayonnement, comprenant :
- enregistrer des données (ID) d'image en utilisant le procédé suivant l'une quelconque des revendications 1 à 5,
- évaluer les données (ID) d'image enregistrées pour déterminer une position (DP) dynamique, à laquelle on peut s'attendre, d'une zone d'exposition au rayonnement d'un patient (P).

7. Dispositif (60) de reconstruction d'image CT comprenant :
- une interface (61) de données brutes pour enregistrer des données (RD) brutes CT d'une zone à examiner d'un patient (P),
- une unité (62) de détection de mouvement de déglutition pour détecter des phases de mouvement différentes d'un mouvement de déglutition du patient (P), dans lequel il est défini une métrique (IM) d'image, qui est sensible et spécifique au mouvement de déglutition et les phases de mouvement différentes sont affectées à des valeurs différentes de la métrique (IM) d'image,
- une unité (63) d'affectation pour déterminer des ensembles de données brutes différents des données (RD) brutes CT enregistrées, affectés à des phases de mouvement détectées différentes sur la base des phases de mouvement détectées, dans lequel les ensembles (RD) de données brutes sont déterminés et affectés à des phases de mouvement différentes sur la base de valeurs différentes de la métrique (IM) d'image,
- une unité (64) de reconstruction pour reconstruire, pour chacun des ensembles de données brutes déterminés, une image (ID) CT individuelle affectée à une phase de mouvement individuelle.

8. Système (1) d'imagerie médicale, comprenant :
- une unité (2) de scan CT pour effectuer une imagerie médicale d'un patient (P),
- un dispositif (60) de reconstruction d'image CT suivant la revendication 7.

9. Dispositif (71) de planification d'un traitement médical par rayonnement, comprenant :
- le système (1) d'imagerie médicale suivant la revendication 8 pour enregistrer des données (ID) d'image en utilisant le procédé suivant l'une quelconque des revendications 1 à 6,
- une unité (71) de planification pour évaluer les données (ID) d'image enregistrées pour déterminer une position (DPO) dynamique à laquelle on peut s'attendre, d'une zone d'un patient (P) exposée à du rayonnement.

10. Système (70) d'un traitement médical par rayonnement, comprenant :
- un dispositif (71) de planification d'un traitement médical par rayonnement suivant la revendication 9,
- une unité (72) de traitement du rayonnement pour soumettre au rayonnement une zone à soumettre au rayonnement d'un patient (P), sur la base de la position (DPO) dynamique déterminée à laquelle on peut s'attendre.

11. Produit de programme d'ordinateur ayant un programme d'ordinateur, qui peut être chargé directement dans un dispositif de mémoire d'un système (1) d'imagerie médicale ou d'un dispositif (71) de planification d'un traitement médical par rayonnement, ayant des parties de programme pour effectuer tous les stades de l'une quelconque des revendications 1 à 6, lorsque le programme d'ordinateur est effectué dans le système (1) d'imagerie médicale ou dans le dispositif (71) de planification d'un traitement médical par rayonnement.

12. Support déchiffrable par ordinateur, sur lequel sont mises en mémoire des parties de programme, qui peuvent être lues et exécutées par une unité informatique afin d'effectuer tous les stades des procédés suivant l'une quelconque des revendications 1 à 6, lorsque les parties de programme sont exécutées par l'unité informatique.
